# EUROPÄISCHE PATENTSCHRIFT

(11) **EP 3 035 895 B1**
(45) Veröffentlichungstag und Bekanntmachung des Hinweises auf die Patenterteilung: **03.01.2018**
(21) Anmeldenummer: 14739355.7
(22) Anmeldetag: 07.07.2014
(51) Int. Cl.: A61F 2/64, A61F 2/68, A61F 5/01

(54) **VERFAHREN ZUR STEUERUNG EINES KÜNSTLICHEN ORTHESEN- ODER PROTHESENKNIEGELENKES**
CONTROL PROCEDURE FOR AN ARTIFICIAL ORTHOTIC OR PROSTHETIC KNEE
PROCÉDURE DE CONTROLE D'UNE ARTICULATION ARTIFICIELLE D'UNE ORTHÈSE OU D'UNE PROTHÈSE DE GENOU

(30) Priorität: 22.08.2013 DE 102013013810
(43) Veröffentlichungstag der Anmeldung: 29.06.2016
(73) Patentinhaber: Otto Bock Healthcare Products GmbH, 1070 Wien (AT)
(72) Erfinder: AUBERGER, Roland, A-1140 Wien (AT); SEYR, Martin, A-1040 Wien (AT); SEIFERT, Dirk, 1070 Wien (AT); MANDL, Clemens, A-1030 Wien (AT); DIETL, Hans, A-3003 Gablitz (AT)
(74) Vertreter: Stornebel, Kai
(86) Internationale Anmeldenummer: PCT/EP2014/001869
(87) Internationale Veröffentlichungsnummer: WO 2015/024612

(56) Entgegenhaltungen:
- WO-A1-2012/100250
- DE-A1- 19 859 931
- US-A1- 2007 010 772

## Beschreibung

Die Erfindung betrifft ein Verfahren zur Steuerung eines künstlichen Orthesen- oder Prothesenkniegelenkes, an dem eine Unterschenkelkomponente angeordnet und der eine Widerstandseinrichtung zugeordnet ist, bei der der Beugewiderstand in Abhängigkeit von Sensordaten verändert wird, die während der Benutzung des Orthesen- oder Prothesenkniegelenkes über zumindest einen Sensor ermittelt werden.

Prothesen- oder Orthesenkniegelenke ersetzen oder unterstützen die Funktion eines natürlichen Kniegelenkes. Um eine möglichst optimale Funktionalität des künstlichen Kniegelenkes zu erreichen, ist eine Vielzahl von Konstruktionen auf dem Markt, die das Verhalten der Kniegelenke während der Standphase und der Schwungphase beeinflussen. Mechatronische Kniegelenke sind bekannt, bei denen über mehrere verschiedene Sensoren die Bewegungssituationen erfasst werden und auf Grundlage der Sensordaten eine Widerstandseinrichtung angesteuert wird, über die der Beugewiderstand oder der Streckwiderstand variiert wird. Eine Grundproblematik besteht darin, dass die große Vielfalt der möglichen Bewegungssituationen sich nur schwer in einfache Regeln fassen lässt. Daher werden zur Steuerung von Aktuatoren und Bremsen sogenannte Zustandsmaschinen eingesetzt, die hochkomplex sind und viele verschiedene Aktivitäten abbilden. Nachteilig hieran sind der lange Entwicklungszeitraum und der Einsatz aufwändiger Bauteile.

Die EP 1 237 513 B1 betrifft eine die Existenz oder Funktion einer Gliedmaße ersetzende Unterstützungsvorrichtung aus mindestens zwei, durch ein künstliches Gelenk miteinander verbundenen Teilen und einer Kontrollvorrichtung. Die Unterstützungsvorrichtung umfasst einen Sensor, der einen Neigungswinkel bezogen auf eine Schwerkraftlinie eines mit dem Gelenk verbundenen Teils erfasst und an die Kontrollvorrichtung gekoppelt ist. Die Kontrollvorrichtung ist so angeordnet, dass sie das Gelenk auf der Basis von vom Sensor übermittelten Neigungswinkeldaten beeinflusst.

Der Neigungswinkelsensor ist bei einer Ausgestaltung als Prothesenkniegelenk an einem Rohrschenkel angeordnet, zur Ergänzung der Datensituation kann ein zweiter Sensor an dem Unterschenkel angeordnet sein.

Die DE 10 2008 027 639 A1 betrifft ein Orthesengelenk zum Unterstützen eines anatomischen Kniegelenks mit einem Gelenkoberteil und einem Gelenkunterteil, die gelenkig miteinander verbunden sind. Ein Sperrelement zum automatischen Entriegeln und Verriegeln des Orthesengelenkes in einer beliebigen Position ist vorgesehen, ebenso wie ein Betätigungselement für das Sperrelement und ein Sensormittel zum Erfassen relevanter Informationen für das Entriegeln und Verriegeln. Eine Auswerteeinheit zum Auswerten der erfassten Informationen und zum Weiterleiten dieser Information an eine Steuerungs- und/oder Regelungseinheit für das Betätigungselement ist ebenfalls vorhanden. Das Sensormittel weist mindestens zwei Sensoren aus der Gruppe Neigungssensoren, Drehwinkelsensoren, Beschleunigungssensoren oder Gyroskope zum Erfassen von Informationen auf, die den Bewegungs- und/oder Ruhezustand eines Menschen beschreiben. Es können zwei Sensoren eines Typs oder je ein Sensor unterschiedlicher Typen ausgewählt werden. Alle Sensoren sind von dem anatomischen Gelenk, insbesondere Kniegelenk, abwärts angeordnet.

Die US 2007/010772 A1 betrifft eine Ortheseneinrichtung mit einer Dämpfereinheit, der Sensoren zugeordnet sind. Die Sensoren sind einmal an der unteren Dämpferaufnahme und einmal im Bereich des Drehgelenks zwischen dem Oberteil und dem Unterteil angeordnet. Über die Sensoren kann die Winkelzuordnung oder die Winkelgeschwindigkeit des Oberteils und des Unterteils relativ zueinander ermittelt werden. Der distale Sensor ist als Beschleunigungssensor ausgestaltet, eine mehrachsige Beschleunigungsmessung ist vorgesehen. Erweiternd ist ausgeführt, dass der Sensor von beliebiger Art und Weise sein kann, sofern er die Möglichkeit einer Ermittlung einer plötzlichen Bewegung des Kniegelenkes bietet. Bei einer Nutzung der Knieorthese bei einem Speedbootfahrer kann es durch die kurzen und harten Beschleunigungen aufgrund der Wellen im Kniegelenk zu Überlastungen kommen. Kniesensoren erfassen die Beschleunigungen, eine elektronische Steuereinheit vergleicht den ermittelten Eingangswert und vergleicht ihn mit einem Schwellwert und erhöht ggf. den Beugewiderstand.

Aufgabe der vorliegenden Erfindung ist es, ein Verfahren zur Steuerung eines künstlichen Orthesen- oder Prothesenkniegelenkes bereitzustellen, mit dem mit einem geringen Steuerungsaufwand ein sicheres Gangverhalten erreicht werden kann.

Erfindungsgemäß wird diese Aufgabe durch ein Verfahren mit den Merkmalen des Hauptanspruches gelöst. Vorteilhafte Ausgestaltungen und Weiterbildungen der Erfindung sind in den Unteransprüchen, der Beschreibung sowie den Figuren offenbart.

Das Verfahren zur Steuerung eines künstlichen Orthesen- oder Prothesenkniegelekes, an dem eine Unterschenkelkomponente angeordnet und der eine Widerstandseinrichtung zugeordnet ist, bei der der Beugewiderstand in Abhängigkeit von Sensordaten verändert wird, die während der Benutzung des Orthesen- oder Prothesenkniegelenkes über zumindest einen Sensor ermittelt werden, sieht vor, dass eine Linearbeschleunigung der Unterschenkelkomponente ermittelt wird und bei Unterschreiten eines Grenzwertes einer Linearbeschleunigung der Unterschenkelkomponente der Beugewiderstand verringert wird. Mit dem vorgestellten Verfahren ist es möglich, eine Steuerung eines Kniegelenkes, unter dem sowohl Orthesenkniegelenke als auch Prothesenkniegelenke verstanden werden, ausschließlich über einfache Sensoren zu realisieren, ohne dass aufwändige und anfällige Kraftmessungen durchgeführt werden müssen. Insbesondere die Verwendung von DMS-Applikationen wird dadurch überflüssig.

Eine Weiterbildung der Erfindung sieht vor, dass eine gestreckte Schrittstellung einer Prothese oder Orthese mit einem Prothesen- oder Orthesenkniegelenk ermittelt wird und bei Vorliegen der gestreckten Schrittstellung der Beugewiderstand verringert wird. Die gestreckte Schrittstellung liegt vor, wenn der Kniewinkel 0° beträgt oder das Kniegelenk leicht eingebeugt ist, also einen Beugewinkel innerhalb eines Bereiches von ±5° aufweist. Liegt die gestreckte Schrittstellung vor, kann davon ausgegangen werden, dass sich der Nutzer des Kniegelenkes in der terminalen Standphase befindet, so dass eine Widerstandsverringerung erfolgen kann.

Zur Detektion einer terminalen Standphase kann zudem ein Absolutwinkel der Unterschenkelkomponente ermittelt und bei Überschreiten eines vorbestimmten Grenzwertes für den Absolutwinkel der Unterschenkelkomponente der Beugewiderstand verringert werden. Über den Neigungswinkel der Unterschenkelkomponente zur Vertikalen lassen sich aussagekräftige Rückschlüsse über die jeweilige Phase innerhalb eines Schrittes ableiten, so dass der Absolutwinkel ein guter Indikator für die Veränderung, insbesondere die Verringerung des Beugewiderstandes ist.

Der Absolutwinkel der Unterschenkelkomponente kann aus einem Absolutwinkel einer Oberschenkelkomponente und einem bekannten, z.B. gemessenen Kniewinkel bestimmt oder direkt mit einem Inertialwinkelsensor gemessen werden, der an der Unterschenkelkomponente befestigt ist.

Weiterhin kann vorgesehen sein, dass zudem über einen Kniewinkelsensor der Kniewinkel ermittelt und bei Unterschreiten eines Grenzwertes für den Kniewinkel Beugewiderstand verringert wird, da der Kniewinkel eine Aussage über der Streckungszustand des Beines oder der Prothese und damit über die jeweilige Phase innerhalb eines Schrittzyklusses zulässt. Der Kniewinkel kann auch aus den Inertialwinkeln des Oberschenkel und des Unterschenkels ermittelt werden.

Liegen mehrere Kenngrößen vor, beispielsweise dass das Überschreiten eines Grenzwertes für den Absolutwinkel der Unterschenkelkomponente, das Unterschreiten eines Grenzwertes für den Kniewinkel und das Unterschreiten eines Grenzwertes einer Beschleunigung der Unterschenkelkomponente, kann mit erhöhter Sicherheit davon ausgegangen werden, dass der Beugewiderstand verändert, insbesondere verringert werden soll, um eine Schwungphasenfreischaltung zu erreichen. Mit dem vorgestellten Verfahren ist es möglich, eine sichere Steuerung eines Kniegelenkes ausschließlich über einfache Sensoren zu realisieren.

Die überwiegende Anzahl mikroprozessorgesteuerter Prothesen- und Orthesensysteme nutzt für die Steuerung eine Kraft- und Momenten-Messung mittels Dehnmessstreifen, wobei als ausschlaggebender Aspekt für die Sicherheit eines Kniegelenks die Umschaltung von einem hohen Beugewiderstand in der Standphase in einen niedrigen Beugewiderstand während der Schwungphase und umgekehrt gilt. Die Umschaltung wird auch als Schwungphasen-Freischaltung bezeichnet. Neben einer Errechnung von Biegemomenten in Knöchelhöhe oder des Kniemomentes müssen für die Freischaltung der Schwungphase Schwellenwerte der aus den Dehnmessstreifen errechneten Werten überschritten werden. Darüber hinaus erfolgt vielfach die Schwungphasen-Freischaltung erst ab einer gewissen Vorwärtsneigung, so dass das Gehen mit kleinen Schritten schwierig wird.

Das erfindungsgemäße Verfahren sieht vor, dass zur Steuerung des Kniegelenks ausschließlich Kniewinkelsensoren und Inertialsensoren verwendet werden, aus deren Daten die notwendigen Größen oder Hilfsgrößen errechnet werden können. Über den Inertialsensor wird der Absolutwinkel der Unterschenkelkomponente ermittelt, also die Neigung der Längserstreckung der Unterschenkelkomponente zur Vertikalen. Der Absolutwinkel muss eine Mindestgröße überschreiten, um eine Vorwärtsneigung, also eine Neigung in Vorwärtsgehrichtung, der Unterschenkelkomponente zu detektieren. Erst ab einem gewissen Winkel in Vorwärtsneigung kann davon ausgegangen werden, dass ein Schritt in Vorwärtsrichtung ausgeführt werden soll. Über einen Kniewinkelsensor wird der Kniewinkel ermittelt. Wird ein Grenzwert für einen Kniewinkel unterschritten, ist dies ein Anzeichen dafür, dass sich der Nutzer in einer terminalen Standphase befindet, so dass eine Freischaltung für die Schwungphase und damit eine Reduzierung des Beugewiderstandes angezeigt ist. Zusätzlich wird die Beschleunigung der Unterschenkelkomponente ermittelt. Wird ein Beschleunigungsgrenzwert unterschritten, kann davon ausgegangen werden, dass sich die Fußkomponente, beispielsweise die Fußplatte oder der Prothesenfuß, noch auf dem Boden befindet, so dass sichergestellt ist, dass der Patient in der terminalen Standphase eines Schrittes befindlich ist und somit die Reduzierung des Beugewiderstandes erfolgen kann.

Eine Weiterbildung der Erfindung sieht vor, dass die Kniewinkelgeschwindigkeit ermittelt wird und erst bei Überschreiten eines Grenzwertes für die Kniewinkelgeschwindigkeit der Beugewiderstand verringert wird. Eine Kniewinkelmindestgeschwindigkeit sollte vorliegen, da anderenfalls eine Stehsituation vorliegen kann, bei der eine Verringerung des Beugewiderstandes unerwünscht sein kann. Über die geringe oder relativ geringe Kniewinkelgeschwindigkeit wird zusätzlich abgesichert, dass sich der Patient in der terminalen Standphase bei einer Vorwärtsbewegung befindet.

Eine Winkelgeschwindigkeit der Unterschenkelkomponente kann berechnet oder über einen Sensor, beispielsweise aus den Sensordaten eines Gyroskops, erfasst werden. Der berechnete oder erfasste Wert der Winkelgeschwindigkeit wird mit einem Grenzwert verglichen und der Beugewiderstand nur dann verringert, wenn die Winkelgeschwindigkeit einen Grenzwert unterschreitet.

Die Linearbeschleunigung der Unterschenkelkomponente wird vorteilhafterweise auf dem Fußsohlenniveau bestimmt oder gemessen und der Steuerung zugrunde gelegt, die Beschleunigung auf Fußsohlenniveau ergibt sich aus den Linearbeschleunigungen an der Position des Beschleunigungssensors, beispielsweise unterhalb der Kniegelenksachse, der Winkelbeschleunigung der Unterschenkelkomponente als Ableitung erster Ordnung eines Gyroskopsignals sowie dem Ortsvektor von der Position des Beschleunigungssensors zu der Bezugsposition am Fuß, beispielsweise am Vorfuß. Über die Linearbeschleunigungen am Fußsohlenniveau werden Rückschlüsse über die kinematische Kontaktbedingung zwischen dem Fuß beziehungsweise Fußteil und dem Untergrund beziehungsweise über die Dynamik der Bewegung gezogen und es kann ermittelt werden, in welcher Phase eines Schrittes der Patient sich befindet. Liegt keine oder nur eine sehr geringe Linearbeschleunigung vor, befindet sich der Fuß, worunter auch ein Prothesenfuß oder ein Fußteil einer Orthese verstanden wird, noch in der Standphase, findet zudem keine Vertikalbeschleunigung mehr statt, ist die Aufsetzphase beendet, so dass über die Linearbeschleunigungen Rückschlüsse auf die Orientierung und Positionierung des Beines gezogen werden können. Aus den Beschleunigungen könne auch entsprechende Geschwindigkeiten ermittelt werden, die ebenfalls zur Steuerung in entsprechender Art und Weise herangezogen werden können.

Eine Weiterbildung der Erfindung sieht vor, dass die Verringerung des Beugewiderstandes bei Vorliegen einer Hyperextension der Unterschenkelkomponente erfolgt, also dass die Hyperextension ein Teil derjenigen Parameter ist, die der Steuerung zur Veränderung des Beugewiderstandes zugrunde gelegt werden. Der Kniewinkel von 0° wird angenommen, wenn die Unterschenkelkomponente kraftlos gegen einen Streckanschlag anliegt. Eine Zunahme des Kniewinkels wird angenommen, wenn eine Einbeugung des Kniegelenkes entgegen der Gehrichtung vorgenommen wird.

Wird eine Überstreckung des Kniegelenks bewirkt, verringert sich der Kniewinkel weiter, da er als negativer Kniewinkel angesehen wird. Erfährt das Kniegelenk ein Extensionsmoment um die Knieachse durch am Gelenk angreifende Kräfte, beispielsweise über die Bodenreaktionskraft, Stumpfkräfte oder Hüftmomente, kann eine Hyperextension auftreten, bei deren Detektion abgeleitet werden kann, dass sich der Patient in der terminalen Standphase befindet und daher eine Schwungphasen-Freischaltung stattfinden soll.

Das Kniegelenk kann einen elastischen Streckanschlag aufweisen, beispielsweise um zu verhindern, dass bei einer Extensionsbewegung die Unterschenkelkomponente hart in den Anschlag einschwenkt. Der elastische Streckanschlag kann aus Elastomerkörpern, Federelementen oder dergleichen bestehen. Durch den elastischen Streckanschlag ist es möglich, bei Aufbringen eines Extensionsmomentes um die Knieachse eine Überstreckung in einem kleinen Winkelbereich zuzulassen, bei Wegfall des Extensionsmomentes wird durch den elastischen Streckanschlag die Unterschenkelkomponente wieder in die gestreckte oder nahezu gestreckte Stellung gebracht, in der der Kniewinkel 0° beträgt. Das Gelenk kehrt nach Wegnahme der Belastung in die gestreckte oder nahezu gestreckte Stellung zurück. Aufgrund der Kenntnis über die Federkennlinie des Streckanschlages und den negativen Kniewinkel ist es möglich, ein Kniemoment, das um die Knieachse in Extensionsrichtung wirkt, zu berechnen und bei Vorliegen eines Kniemomentes in Extensionsrichtung, also bei einer Kompression des elastischen Streckanschlages, den Beugewiderstand zu verringern. Das Kniemoment wird dabei auf der Grundlage des Kniewinkels und der Kenntnis der Federkennlinie errechnet, Kraftmesssensoren oder Momentensensoren sind nicht notwendig. Liegt ein Kniemoment in Extensionsrichtung vor, ist dies ein weiterer Faktor zur Beurteilung, in welcher Phase eines Schrittes sich der Patient befindet und ob eine Schwungphase unmittelbar folgt und dementsprechend der Beugewiderstand verringert werden soll.

Eine Rotationsrichtung der Unterschenkelkomponente kann berechnet oder über einen Sensor, beispielsweise einen Gyroskopen, erfasst werden. Der Beugewiderstand wird nur dann verringert, wenn eine Vorwärtsrotation der Unterschenkelkomponente vorliegt, um auszuschließen, dass bei einem Rückwärtsgehen der Beugewiderstand ungewollt verringert wird.

Über einen Beschleunigungssensor können Beschleunigungsdaten der Unterschenkelkomponente ermittelt werden, um benötigte Kenngrößen ableiten oder berechnen zu können.

Nach einer Verringerung des Beugewiderstandes kann dieser wieder erhöht werden, insbesondere auf ein Standphasenniveau oder auf ein so hohes Niveau, dass ein ungewolltes Einbeugen nicht oder nur verlangsamt möglich wird, wenn innerhalb eines vordefinierten Zeitrahmens keine Einbeugung des Kniegelenkes erfolgt oder innerhalb eines Beugewinkels nach erfolgter Einbeugung ein vorher festgelegter Grenzwert für eine Horizontalbeschleunigung überschritten wird. Dies dient dazu, die Sicherheit zu erhöhen, wenn eine Schwungphase abgebrochen wird, also kein vollständiger Schrittzyklus vorgenommen werden kann.

Nachfolgend wird ein Ausführungsbeispiel der Erfindung anhand der Figuren näher erläutert. Es zeigen:
- Figur 1 -: eine schematische Darstellung eines Prothesenkniegelenkes;
- Figur 2 -: eine schematische Darstellung eines Streckanschlages;
- Figur 3 -: ein Beispiel eines Steuerungsablaufes; sowie
- Figur 4 -: eine schematische Darstellung eines Steuerungskonzeptes.

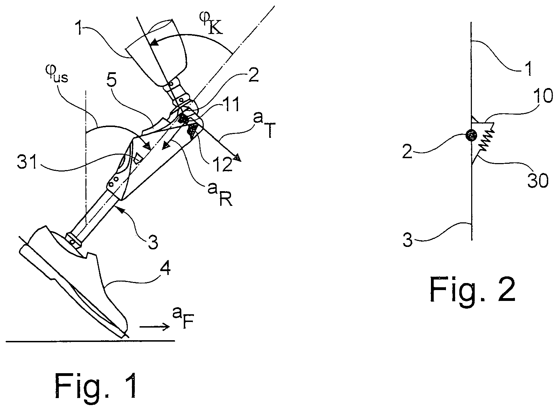

In der Figur 1 ist eine Protheseneinrichtung für Patienten mit einem Oberschenkelstumpf und fehlendem Kniegelenk und Unterschenkel dargestellt. Ein Prothesenschaft 1, auch als Oberschenkelkomponente der Prothese oder Oberschenkelschaft zu bezeichnen, dient zur Aufnahme des nicht dargestellten Stumpfes. An dem Prothesenschaft 1 ist ein Prothesenkniegelenk 2 angeordnet, das im vorliegenden Fall als monozentrisches Kniegelenk ausgebildet ist und um eine Schwenkachse herum eine Unterschenkelkomponente 3 verschwenkbar zu dem Oberschenkelschaft 1 lagert. An dem distalen Ende der Unterschenkelkomponente 3 ist ein Prothesenfuß 4 angeordnet. Die Protheseneinrichtung ist in einer terminalen Standphase dargestellt, der Prothesenfuß 4 befindet sich noch auf dem Boden. Innerhalb der Unterschenkelkomponente 3 ist eine Widerstandseinrichtung 5 angeordnet, die einer Einbeugung, also Flexion, einen Widerstand entgegensetzt, ebenso dient die Widerstandseinrichtung 5 zur variablen Einstellung eines Streckwiderstandes, also eines Extensionswiderstandes. Der Widerstand innerhalb der Widerstandseinrichtung wird über einen Aktuator verändert, der beispielsweise Ventile öffnet oder schließt oder Hydraulikströme umleitet. Alternativ ist es ebenso möglich, dass der Aktuator rheologische Eigenschaften des Hydraulikfluids verändert, um den Widerstand zu verändern. Alternative Widerstandsveränderungen sind möglich, beispielsweise die Aktivierung von Bremsen oder die Umwandlung von Bewegungsenergie in elektrische Energie.

An der Unterschenkelkomponente 3 ist ein Inertialsensor 31 angeordnet, der den Absolutwinkel ϕᵤₛ der Unterschenkelkomponente aufzeichnet. Der Inertialsensor 31 misst den Absolutwinkel ϕᵤₛ der Unterschenkelkomponente 3 gegenüber der Vertikalen und kann als 2D- oder 3D-Magnetfeldsensor, 2D- oder 3D-Beschleunigungssensor oder als Gyroskop ausgebildet sein. Der Absolutwinkel ϕᵤₛ vergrößert sich bei zunehmender Neigung der Unterschenkelkomponente 3 in Vorwärtsgehrichtung, also bei einer Verschwenkung um einen distalen Kontaktpunkt mit dem Boden in Uhrzeigerrichtung. Weiterhin ist an der Unterschenkelkomponente 3 ein Beschleunigungssensor 12 angeordnet, über den eine Tangentialbeschleunigung a_{T}, also eine Beschleunigung tangential zum Verschwenkradius der Unterschenkelkomponente 3, und eine Radialbeschleunigung a_{R}, also eine Beschleunigung in Richtung auf den distalen Drehpunkt der Unterschenkelkomponente 3, des Kniegelenks 12 ermittelt werden kann. Es ist auch möglich, mit einem entsprechenden Sensor, z.B. einem 3D-Beschleunigungssensor zusätzlich die Medial- und Lateralbeschleunigung zu erfassen oder nur diese Beschleunigungen.

Schließlich ist ein Kniewinkelsensor 11 vorgesehen, über den der Kniewinkel ϕₖ erfasst werden kann. Der Kniewinkel ϕₖ wird von der Verlängerung der Längserstreckung der Unterschenkelkomponente 3 in Beugerichtung positiv zunehmend betrachtet, der Kniewinkel ϕₖ ist 0, wenn die Längserstreckung des Prothesenschaftes 1 mit der Achse der Längserstreckung der Unterschenkelkomponente 3 fluchtet. Eine Hyperextension, also eine Überstreckung in Extensionsrichtung, wird als negativer Kniewinkel ϕₖ angesehen.

Das Prothesenkniegelenk 2 kann einen elastischen Streckanschlag aufweisen, der schematisch in der Figur 2 dargestellt ist. Neben dem schematischen Oberschenkelschaft 1 und der schematischen Unterschenkelkomponente 3, die verschwenkbar aneinander um eine Gelenkachse gelagert sind, ist ein Widerlager 10 an dem Oberteil des Prothesenkniegelenkes 2 angeordnet. Das Widerlager 10 ist im Wesentlichen starr ausgebildet und im gestreckten Zustand, wie in der Figur 2 dargestellt ist, liegt ein elastisches Anschlagelement 30 an dem starren Widerlager 10 an. Eine geringfügige Hyperextension wird durch die Federausgestaltung ermöglicht, über die Kenntnis der Federrate des Anschlagselementes 30 kann das in Extensionsrichtung wirkende Kniemoment aus dem Kniewinkel ϕₖ errechnet werden. Selbstverständlich ist es auch möglich, das Widerlager 10 an der Unterschenkelkomponente und das elastische Anschlagelement an dem Oberteil 1 anzuordnen.

Für die Steuerung einer Schwungphasen-Freischaltung können mehrere Kenngrößen herangezogen werden, nämlich einmal die Vorwärtsneigung der Unterschenkelkomponente 3, also ein positiver Unterschenkelwinkel ϕᵤₛ der Unterschenkelkomponente 3, eine Vorwärtsrotation des Unterschenkels in Gehrichtung, also eine Zunahme des Absolutwinkel ϕᵤₛ der Unterschenkelkomponente 3, eine Beschleunigung des Kniegelenks, um insbesondere den Bewegungszustand des Prothesenfußes 4 auf Fußsohlenniveau zu bestimmen sowie den Kniewinkel ϕₖ und eine Kniewinkelgeschwindigkeit ω_{K}, die aus der ersten zeitlichen Ableitung des Kniewinkels ϕₖ errechnet werden kann.

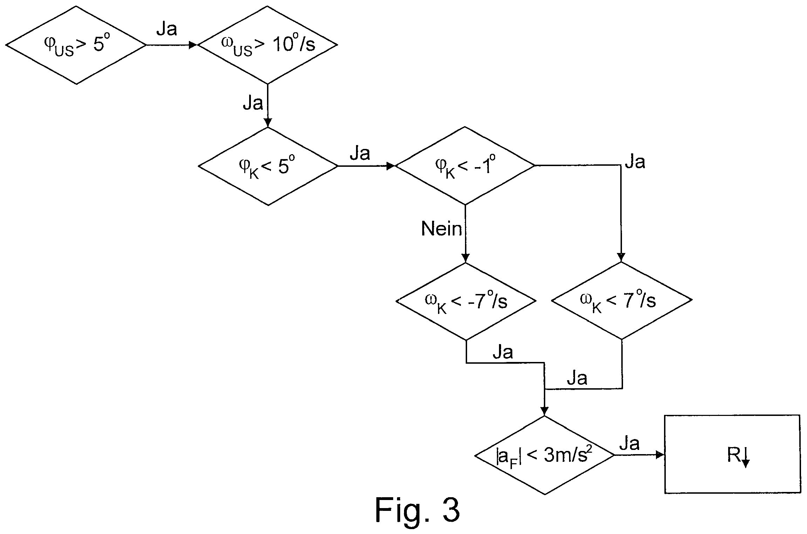

In der Figur 3 ist ein schematischer Ablauf der Steuerung gezeigt. Um eine Schwungphase freizuschalten und den Beugewiderstand R der Widerstandseinrichtung 5 zu verringern, wird zunächst die Vorwärtsneigung, also der positive Absolutwinkel ϕᵤₛ der Unterschenkelkomponente 3 relativ zur Vertikalen bestimmt. Liegt der Absolutwinkel ϕᵤₛ oberhalb eines festgelegten Grenzwertes, beispielsweise 5°, ist die erste Bedingung für eine Schwungphasen-Freischaltung erreicht. Wird darüber hinaus noch eine Vorwärtsrotation in Gestalt einer Unterschenkelwinkelgeschwindigkeit ωᵤₛ detektiert, kann angenommen werden, dass sich der Unterschenkel in einer Bewegung befindet, als Grenzwert kann beispielsweise eine Vorwärtsrotation > 10°/s angenommen werden. Sobald diese Grenzwerte erfüllt oder überschritten sind, wird überprüft, ob der Kniewinkel ϕₖ einem festgelegten Grenzwert entspricht. In der terminalen Standphase, die vor der Einleitung der Schwungphase eingenommen wird, befindet sich das Prothesenkniegelenk 2 in einer gestreckten oder nahezu gestreckten Position, bei einem elastischen Streckanschlag kann sogar eine Hyperextension stattfinden. Wird als Kniewinkel ϕₖ ein Grenzwert unterschritten, der < 5° ist und auch negative Werte annehmen kann, dann liegt eine weitere Bedingung für das Einleitung einer Schwungphasen-Freischaltung vor. Über den Kniewinkel ϕₖ und die bekannten Daten der Federeinrichtung in dem elastischen Streckanschlag kann das in Extensionsrichtung wirksame Kniemoment errechnet werden.

Liegt ein negativer Kniewinkel ϕₖ vor, liegt also eine Hyperextension vor, wird überprüft, wie groß die Kniewinkelgeschwindigkeit ω_{K} ist. Liegt diese unterhalb eines Grenzwertes, beispielsweise unter 7°/s, kann angenommen werden, dass keine oder nur eine geringe Kniebeugung und Kniedynamik vorhanden ist, was ebenfalls charakteristisch für eine terminale Standphase ist. Liegt keine Hyperextension vor, wird überprüft, ob die negative Winkelgeschwindigkeit einen Grenzwert unterschreitet, so dass hier abgefragt wird, wie groß die Kniewinkelgeschwindigkeit in Flexionsrichtung oder Extensionsrichtung ist. Liegen die ermittelten Kniewinkelgeschwindigkeiten ω_{K} unterhalb der geforderten Grenzwerte, wird der Betrag der Beschleunigung a_{F} auf Fußsohlenniveau errechnet, der sich aufgrund der relativen Lage des Beschleunigungsvektors zum Prothesenfußes 4 ergibt. Liegt die Beschleunigung a_{F} auf Fußsohlenniveau unter einem Grenzwert, beispielsweise unterhalb 3m/s², ist davon auszugehen, dass die kinematischen Kontaktbedingungen zwischen dem Prothesenfuß 4 und dem Untergrund denen einer terminalen Standphase entsprechen und somit die Reduzierung des Widerstandes R der Widerstandseinrichtung 5 eingeleitet werden kann.

Die eindeutige Entscheidung zwischen einem Abrollen über den Prothesenfuß 4 nach vorne, also einem Vorwärtsschritt, und einem Durchschwingen der Prothese unter dem Körper nach hinten, zum Beispiel in der Schwungphase eines Rückwärtsschrittes, sind all diejenigen Schritte und Abfragen notwendig, die sich nach der Feststellung einer Vorwärtsneigung und Vorwärtsrotation der Unterschenkelkomponente 3 anschließen. Dazu wird eine Hyperextension des Prothesenkniegelenkes 2 entgegen einem elastischen Extensionsanschlag 10, 30 oder eine stark streckende Bewegung bei einem geringen Kniewinkel ϕₖ verlangt, die durch den Kniewinkelsensor 11 gemessen werden kann. Zusätzlich wird über den Beschleunigungssensor 12 ermittelt, ob das Extensionsmoment um das Kniegelenk statisch oder dynamisch aufgebracht wird. Dabei werden insbesondere die Linearbeschleunigungen der Prothese auf dem Fußsohlenniveau berechnet. Unter der Voraussetzung, dass die Vorwärtsneigung, also der positive Absolutwinkel ϕᵤₛ, und eine Vorwärtsrotation der Unterschenkelkomponente gegeben sind, lassen sich anhand von Beschleunigungen und Kniemomenten Fallunterscheidungen vornehmen, auf deren Grundlage der Beugewiderstand R entweder auf einem hohen Standphasenflexionsniveau beibehalten wird oder auf ein Schwungphasenniveau reduziert wird.

Liegt um das Prothesenkniegelenk 1 kein ausreichendes Extensionsmoment oder ein ausreichendes Beugemoment vor oder ist die Kniewinkelgeschwindigkeit ω_{K} entweder streckend oder befindet sich das Prothesenkniegelenk 2 in Hyperextension, kann keine Schwungsphasen-Freischaltung erfolgen.

Erfährt das Prothesenkniegelenk ein Extensionsmoment um die Knieachse aufgrund dynamischer Kräfte, beispielsweise aufgrund der Trägheitskräfte des Prothesenfußes 4 und der Unterschenkelkomponente 3, kann eine Hyperextension im Kniegelenk oder eine streckende Bewegung gemessen werden. Bewegt sich somit die Unterschenkelkomponente 3, entspricht diese Situation der eines Pendels, so dass keine Schwungphasen-Freischaltung erfolgt.

Eine Schwungphasen-Freischaltung erfolgt entsprechend dann, wenn ein Extensionsmoment um die Knieachse durch am Prothesenkniegelenk angreifende statische Kräfte, wie Bodenreaktionskraft und Stumpfkräfte, hervorgerufen wird. In diesem Fall wird eine Hyperextension oder eine stark streckende Bewegung des Kniegelenkes gemessen, jedoch keine oder nur eine geringfügige Beschleunigung a_{F} auf Fußsohlenniveau. Eine solche Situation ist charakteristisch für die terminale Standphase, in der die Prothese unter Belastung in Gehrichtung über den Fuß abrollt. In dieser Situation wird der Widerstand R reduziert.

Die Charakteristik der elastischen Hyperextension, insbesondere der Federkennlinie des elastischen Streckanschlages, sowie die Schwellwerte für den Kniewinkel ϕₖ, die Kniewinkelgeschwindigkeit ω_{K} und die zulässigen Beschleunigungen a_{F} zur Schwungphasen-Freischaltung müssen so gewählt werden, dass einerseits eine eindeutige Unterscheidung vorgenommen werden kann, ob eine Schwungphasen-Freischaltung erfolgt und andererseits auch bei einer geringen Hyperextension, beispielsweise von Anwendern mit einem geringen Körpergewicht und bei kleinen Schritten und langsamen Gehgeschwindigkeiten erreicht wird.

Sollte beispielsweise innerhalb der ersten 5° einer Knieeinbeugebewegung nach einer Freischaltung der Widerstandseinrichtung 5 in einen verringerten Beugewiderstand R eine Beschleunigung a_{F} betragsmäßig oberhalb einer definierten Schwelle festgestellt werden, beispielsweise durch Anstoßen an ein Hindernis, kann der Beugewiderstand R umgehend wieder in eine hohe Standphasen-Flexionsdämpfung umgeschaltet werden, um ein ungewolltes Einbeugen in einem Notfall zu vermeiden.

Sämtliche gemessenen Signale der Sensoren können gefiltert werden, um Messungenauigkeiten ausgleichen zu können. Für die Beschleunigungsbedingungen können asymmetrische Grenzwerte festgelegt werden, um eine individuelle Anpassung an die jeweiligen Gangsituation und Bewegungsrichtungen vornehmen zu können.

Die vorgestellte Steuerung kommt ohne direkte Kraftmessung aus und kann somit auf gegebenenfalls empfindliche und schwer auszuwertende Kraftsensoren verzichten. Als Sensoren werden ausschließlich Kniewinkelsensor, Inertialwinkelsensoren, wie Gyroskope und Beschleunigungssensoren verwendet. Mit diesen Sensoren können die Momentenverhältnisse um die Knieachse, insbesondere in Extensionsrichtung, einfach ermittelt werden, indem die Kenngrößen eines elastischen Streckanschlages erfasst und der Berechnung zugrunde gelegt werden.

Die einfach errechenbaren Linear- und Winkelbeschleunigungen werden zur Berechnung des Bewegungszustandes der Prothese herangezogen, insbesondere um den Bewegungszustand des Prothesenfußes 4 zu identifizieren. Durch die logische Verknüpfung von Kräften und Momenten mit Beschleunigungen kann zwischen statischen Kräften und dynamischen Kräften und Momenten unterschieden werden, so dass über diese Unterscheidung eine Detektion über das Gangverhalten erreicht werden kann. Eine Unterscheidung, ob ein freies Durchschwingen oder eine terminale Standphase vorliegt, kann auf diese Art und Weise leicht getroffen werden.

Die Steuerung nach dem oben beschriebenen Verfahren ermöglicht zudem das zuverlässige Freischalten in die Schwungphase auch bei langsamen Gehgeschwindigkeiten, kleinen Schritten und bei weichen Untergründen, wie lockerem Sand oder Schnee. Die Steuerung ist unabhängig von dem Patientengewicht und kann ein sicheres Rückwärtsgehen gewährleisten.

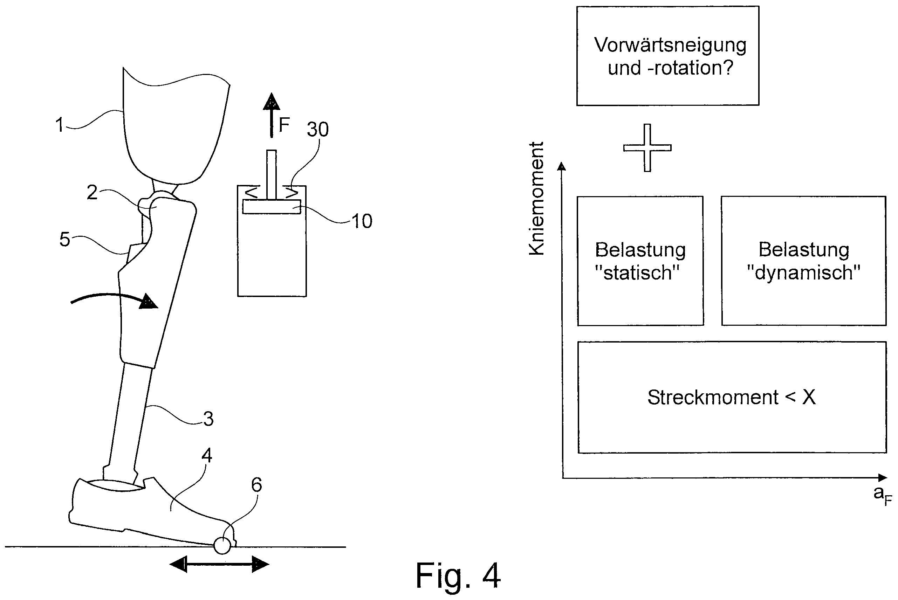

In der Figur 4 ist eine schematische Darstellung eines Steuerungskonzeptes eines Prothesenkniegelenkes dargestellt, der konstruktive Aufbau der Prothese entspricht der der Figur 1, grundsätzlich ist es auch möglich, das Steuerungskonzept bei Orthesen, insbesondere einer sogenannten KAFO (knee ankle foot orthosis) einzusetzen. Der Prothesenschaft 1 oder die Oberschenkelkomponente ist über das Prothesenkniegelenk 2 mit der Unterschenkelkomponente 3 verbunden. An dem distalen Ende der Unterschenkelkomponente 3 ist der Prothesenfuß 4 angeordnet. Innerhalb der Unterschenkelkomponente 3 befindet sich ebenfalls die Widerstandseinrichtung 5. Der elastische Streckanschlag, der insbesondere innerhalb der Widerstandseinrichtung 5 angeordnet sein kann, ist rechts neben der schematischen Darstellung der Protheseneinrichtung gezeigt. Die Protheseneinrichtung befindet sich in der gestreckten Schrittstellung in der terminalen Standphase, was bedeutet, dass im Vorderfußbereich ein Auflage- und Drehpunkt 6 entsteht, um den die Protheseneinrichtung rotiert. Aufgrund des Hebels zwischen der Längsachse der Unterschenkelkomponente 3 und dem Auflagepunkt 6 wird ein Extensionsmoment um das Kniegelenk 2 herum aufgebracht, was dazu führt, dass das in diesem Fall bewegliche Widerlager 10 als Teil eines Hydraulikkolbens gegen das Anschlagelement 30 in Gestalt einer Feder gedrückt wird. Gleichzeitig wird erfasst, ob die Protheseneinrichtung eine Vorwärtsneigung aufweist, was durch den gebogenen Pfeil angedeutet ist. Liegt ein Absolutwinkel ϕᵤₛ vor, im dargestellten Ausführungsbeispiel also eine Neigung in Uhrzeigerrichtung zur Vertikalen, und findet eine Vorwärtsrotation statt, also eine Zunahme des Absolutwinkels ϕᵤₛ in Vorwärtsgehrichtung, wobei die Rotation um den distalen Auflagepunkt 6 erfolgt, sind weitere Kriterien zur Bestimmung des momentanen Zustandes und der Phase innerhalb eines Schrittzyklusses oder eines Schrittablaufes gegeben. Das Extensionsmoment kann durch die Federrate des Anschlagelementes 30 und den Kniewinkel, in diesem Fall den negativen Kniewinkel ϕ_{K}, errechnet werden.

Um auszuschließen, dass die Unterschenkelkomponente nur frei um das Kniegelenk 2 schwingt, wird die Linearbeschleunigung a_{F} im Auflagepunkt 6 ermittelt. Ist diese Beschleunigung 0 oder sehr gering, kann davon ausgegangen werden, dass der Prothesenfuß 4 und der Auflagepunkt 6 Bodenkontakt haben, so dass ein stationärer Drehpunkt im Auflagepunkt 6 vorhanden ist. Die Belastung ist quasi statisch. Bei einer statischen Belastung, einer Vorwärtsneigung und Vorwärtsrotation und gegebenenfalls Hyperextension, wenn das Kniemoment Streckmoment einen Grenzwert X nicht überschreitet, wird dann der Beugewiderstand R der Widerstandseinrichtung 5 verringert, so dass ein Einbeugen des Prothesenkniegelenkes 2 leicht erfolgen kann.

## Patentansprüche

1. Verfahren zur Steuerung eines künstlichen Orthesen- oder Prothesenkniegelenkes (2), an dem eine Unterschenkelkomponente (3) angeordnet und der eine Widerstandseinrichtung (5) zugeordnet ist, bei der der Beugewiderstand R in Abhängigkeit von Sensordaten verändert wird, die während der Benutzung des Orthesen- oder Prothesenkniegelenkes über zumindest einen Sensor (11, 12, 31) ermittelt werden, wobei eine Linearbeschleunigung a_{F} der Unterschenkelkomponente (3) ermittelt und die ermittelte Linearbeschleunigung a_{F} mit zumindest einem Schwellwert verglichen wird, **dadurch gekennzeichnet, dass** bei Unterschreiten eines Grenzwertes der Linearbeschleunigung a_{F} der Unterschenkelkomponente (3) der Beugewiderstand R verringert wird.

2. Verfahren nach Anspruch 1, **dadurch gekennzeichnet, dass** eine gestreckte Schrittstellung einer Prothese oder Orthese mit einem Prothesen- oder Orthesenkniegelenk ermittelt wird und bei Vorliegen der gestreckten Schrittstellung der Beugewiderstand R verringert wird.

3. Verfahren nach Anspruch 1 oder 2, **dadurch gekennzeichnet, dass** zur Detektion einer terminalen Standphase ein Absolutwinkel ϕᵤₛ der Unterschenkelkomponente (3) ermittelt wird und bei Überschreiten eines vorbestimmten Grenzwertes für den Absolutwinkel ϕᵤₛ der Unterschenkelkomponente (3) der Beugewiderstand R verringert wird.

4. Verfahren nach Anspruch 3, **dadurch gekennzeichnet, dass** der Absolutwinkel ϕᵤₛ der Unterschenkelkomponente (3) aus einem Absolutwinkel einer Oberschenkelkomponente und einem Kniewinkel ϕₖ oder direkt mit einem Inertialwinkelsensor (31) gemessen wird.

5. Verfahren nach einem der voranstehenden Ansprüche, **dadurch gekennzeichnet, dass** über einen Kniewinkelsensor (11) ein Kniewinkel ϕₖ ermittelt und bei Unterschreiten eines vorbestimmten Grenzwertes für den Kniewinkel ϕₖ der Beugewiderstand R verringert wird.

6. Verfahren nach einem der voranstehenden Ansprüche, **dadurch gekennzeichnet, dass** zusätzlich eine Kniewinkelgeschwindigkeit ω_{K} ermittelt und erst bei Überschreiten eines Grenzwertes der Beugewiderstand R verringert wird.

7. Verfahren nach einem der voranstehenden Ansprüche, **dadurch gekennzeichnet, dass** eine Winkelgeschwindigkeit ωᵤₛ der Unterschenkelkomponente (3) berechnet oder über einen Sensor (12) erfasst und der Beugewiderstand R nur dann verringert wird, wenn die Winkelgeschwindigkeit ωᵤₛ einen Grenzwert unterschreitet.

8. Verfahren nach einem der voranstehenden Ansprüche, **dadurch gekennzeichnet, dass** die Linearbeschleunigung a_{F} der Unterschenkelkomponente (3) auf dem Fußsohlenniveau der Steuerung zugrunde gelegt wird.

9. Verfahren nach einem der voranstehenden Ansprüche, **dadurch gekennzeichnet, dass** die Verringerung des Beugewiderstandes R bei Vorliegen einer Hyperextension der Unterschenkelkomponente (3) erfolgt.

10. Verfahren nach einem der voranstehenden Ansprüche, **dadurch gekennzeichnet, dass** das Kniegelenk (2) einen elastischen Streckanschlag (10, 30) aufweist, ein Kniemoment über den Kniewinkel ϕₖ und eine Federkennlinie des Streckanschlages berechnet wird und der Beugewiderstand R verringert wird, wenn ein Kniemoment in Extensionsrichtung einen Schwellwert überschreitet.

11. Verfahren nach einem der voranstehenden Ansprüche, **dadurch gekennzeichnet, dass** eine Rotationsrichtung der Unterschenkelkomponente (3) berechnet oder über einen Sensor erfasst und der Beugewiderstand R nur dann verringert wird, wenn eine Vorwärtsrotation vorliegt.

12. Verfahren nach einem der voranstehenden Ansprüche, **dadurch gekennzeichnet, dass** über einen Beschleunigungssensor (12) und/oder Inertialwinkelensor (31) Beschleunigungsdaten der Unterschenkelkomponente (3) ermittelt werden.

13. Verfahren nach einem der voranstehenden Ansprüche, **dadurch gekennzeichnet, dass** nach einer Verringerung des Beugewiderstandes R dieser wieder erhöht wird, wenn innerhalb eines vordefinierten Zeitrahmens keine Einbeugung des Kniegelenkes (2) erfolgte oder innerhalb eines eingenommen Kniewinkels ϕₖ ein Grenzwert für eine Beschleunigung überschritten wird.

## Claims

1. A method for controlling an artificial orthotic or prosthetic knee joint (2), on which a below-knee component (3) is arranged and which is assigned a resistance device (5) in which the flexion resistance R is changed in accordance with sensor data that are determined via at least one sensor (11, 12, 31) during the use of the orthotic or prosthetic knee joint, wherein a linear acceleration a_{F} of the below-knee component (3) is determined and the determined linear acceleration a_{F} is compared with at least one threshold value, **characterized in that**, if a limit value of the linear acceleration a_{F} of the below-knee component (3) is not reached, the flexion resistance R is reduced.

2. The method as claimed in claim 1, **characterized in that** an extended stride position of a prosthesis or orthosis with a prosthetic or orthotic knee joint is determined, and, when the extended stride position is present, the flexion resistance R is reduced.

3. The method as claimed in claim 1 or 2, **characterized in that**, in order to detect a terminal stance phase, an absolute angle ϕ_{US} of the below-knee component (3) is determined, and, if a predefined limit value for the absolute angle ϕ_{US} of the below-knee component (3) is exceeded, the flexion resistance R is reduced.

4. The method as claimed in claim 3, **characterized in that** the absolute angle ϕ_{US} of the below-knee component (3) is measured from an absolute angle of a thigh component and a knee angle ϕ_{K} or directly with an inertial angle sensor (31).

5. The method as claimed in one of the preceding claims, **characterized in that** a knee angle ϕ_{K} is determined via a knee angle sensor (11), and, if a predefined limit value for the knee angle ϕ_{K} is not reached, the flexion resistance R is reduced.

6. The method as claimed in one of the preceding claims, **characterized in that** a knee angle velocity ω_{K} is additionally determined, and the flexion resistance R is reduced only when a limit value is exceeded.

7. The method as claimed in one of the preceding claims, **characterized in that** an angular velocity ω_{US} of the below-knee component (3) is calculated or is detected via a sensor (12), and the flexion resistance R is reduced only when the angular velocity ω_{US} is below a limit value.

8. The method as claimed in one of the preceding claims, **characterized in that** the linear acceleration a_{F} of the below-knee component (3) at the sole level is used as a basis for the control.

9. The method as claimed in one of the preceding claims, **characterized in that** the reduction of the flexion resistance R takes place when there is a hyperextension of the below-knee component (3).

10. The method as claimed in one of the preceding claims, **characterized in that** the knee joint (2) has an elastic extension stop (10, 30), a knee moment is calculated via the knee angle ϕ_{K} and a spring characteristic of the extension stop is calculated, and the flexion resistance R is reduced if a knee moment in the extension direction exceeds a threshold value.

11. The method as claimed in one of the preceding claims, **characterized in that** a rotation direction of the below-knee component (3) is calculated or is detected via a sensor, and the flexion resistance R is reduced only if there is a forward rotation.

12. The method as claimed in one of the preceding claims, **characterized in that** acceleration data of the below-knee component (3) are determined via an acceleration sensor (12) and/or inertial angle sensor (31).

13. The method as claimed in one of the preceding claims, **characterized in that**, after a reduction of the flexion resistance R, the latter is increased again if, within a predefined time interval, no bending of the knee joint (2) took place, or if, within an enclosed knee angle ϕ_{K}, a limit value for an acceleration is exceeded.

## Revendications

1. Procédé de commande d'une articulation de genou orthétique ou prothétique artificielle (2) sur laquelle est agencé un composant formant jambe (3) et à laquelle est associé un dispositif de résistance (5) dans lequel la résistance à la flexion (R) est modifiée en fonction de données de capteur qui sont détectées par au moins un capteur (11, 12, 31) pendant l'utilisation de l'articulation de genou orthétique ou prothétique,
dans lequel on détermine une accélération linéaire a_{F} du composant formant jambe (3) et on compare l'accélération linéaire déterminée a_{F} à au moins une valeur seuil,
**caractérisé en ce que** lors d'un passage au-dessous d'une valeur limite de l'accélération linéaire a_{F} du composant formant jambe (3), la résistance à la flexion R est réduite.

2. Procédé selon la revendication 1, **caractérisé en ce que** l'on détermine une position en extension de marche d'une prothèse ou d'une orthèse pourvue d'une articulation de genou prothétique ou orthétique, et lorsque la position en extension de marche se présente, la résistance à la flexion R est réduite.

3. Procédé selon la revendication 1 ou 2, **caractérisé en ce que** pour la détection d'une phase terminale en station debout, on détermine un angle absolu ϕᵤₛ du composant formant jambe (3), et lors d'un passage au-dessus d'une valeur limite prédéterminée pour l'angle absolu ϕᵤₛ du composant formant jambe (3), la résistance à la flexion R est réduite.

4. Procédé selon la revendication 3, **caractérisé en ce que** l'on mesure l'angle absolu ϕᵤₛ du composant formant jambe (3) à partir d'un angle absolu d'un composant formant cuisse et d'un angle de genou ϕₖ ou directement au moyen d'un capteur d'angle inertiel (31).

5. Procédé selon l'une des revendications précédentes, **caractérisé en ce que** l'on détermine un angle de genou ϕₖ au moyen d'un capteur d'angle de genou (11) et lors d'un passage au-dessous d'une valeur limite prédéterminée pour l'angle de genou ϕₖ, la résistance à la flexion R est réduite.

6. Procédé selon l'une des revendications précédentes, **caractérisé en ce que** l'on détermine en supplément une vitesse angulaire de genou ωₖ et uniquement lors d'un passage au-dessus d'une valeur limite, la résistance à la flexion R est réduite.

7. Procédé selon l'une des revendications précédentes, **caractérisé en ce que** l'on calcule une vitesse angulaire ωᵤₛ du composant formant jambe (3) ou on la détecte au moyen d'un capteur (12), et la résistance à la flexion R n'est réduite que lorsque la vitesse angulaire ωᵤₛ passe au-dessous d'une valeur limite.

8. Procédé selon l'une des revendications précédentes, **caractérisé en ce que** pour la commande, on prend pour base l'accélération linéaire a_{F} du composant formant jambe (3) au niveau de la plante du pied.

9. Procédé selon l'une des revendications précédentes, **caractérisé en ce que** la réduction de la résistance à la flexion R s'effectue en présence d'une hyper-extension du composant formant jambe (3).

10. Procédé selon l'une des revendications précédentes, **caractérisé en ce que** l'articulation de genou (2) comprend une butée d'extension élastique (10, 30), un couple de genou sur l'angle de genou ϕk et une courbe caractéristique d'élasticité de la butée d'extension, et la résistance à la flexion R est réduite lorsqu'un couple de genou dépasse une valeur seuil en direction d'extension.

11. Procédé selon l'une des revendications précédentes, **caractérisé en ce que** l'on calcule une direction de rotation du composant formant jambe (3) ou on la détecte par un capteur, et la résistance à la flexion R n'est réduite qu'en présence d'une rotation vers l'avant.

12. Procédé selon l'une des revendications précédentes, **caractérisé en ce que** l'on détermine des données d'accélération du composant formant jambe (3) par un capteur d'accélération (12) et/ou par un capteur d'angle inertiel (31).

13. Procédé selon l'une des revendications précédentes, **caractérisé en ce qu'**après une réduction de la résistance à la flexion R, celle-ci est de nouveau augmentée lorsqu'à l'intérieur d'une fenêtre de temps prédéterminée, il ne se produit pas de flexion de l'articulation de genou (2) ou lorsqu'une valeur limite pour une accélération est dépassée à l'intérieur d'un angle de genou ϕₖ occupé.
